# EUROPEAN PATENT SPECIFICATION

(11) **EP 0 774 986 B1**
(45) Date of publication and mention of the grant of the patent: **16.02.2000**
(21) Application number: 95924415.3
(22) Date of filing: 15.06.1995
(51) Int. Cl.: A61M 15/00

(54) **INHALER APPARATUS WITH OPTIMISATION CHAMBER**
INHALATOR MIT OPTIMIERUNGSKAMMER
INHALATEUR A CHAMBRE D'OPTIMISATION

(30) Priority: 16.08.1994 GB 9416486
(43) Date of publication of application: 28.05.1997
(73) Proprietor: NORTON HEALTHCARE LIMITED, Harlow Essex CM19 5TJ (GB)
(72) Inventor: BELL, John, H., Loughborough Leicestershire LE11 3JR (GB)
(74) Representative: Watkins, David
(86) International application number: GB9501390
(87) International publication number: WO9604948

(56) References cited:
- WO-A-92/20391
- WO-A-93/11817
- WO-A-93/18811
- WO-A-94/17369
- US-A- 3 456 645

## Description

The present invention relates to a medicament dispensing device according to the preamble of claim 1.

A standard metered dose inhaler consists of three main parts: a pressurised canister, an actuator body and a drug delivery outlet. The pressurised canister contains a mixture of active drug and propellant and is usually formed from a deep drawn aluminium cup portion having a lid portion crimped thereto which carries the metering valve assembly. The metering valve assembly is provided with a protruding valve stem which, in use, is inserted as a tight push fit into a so-called "stem block" in the actuator body.

Operation of the inhaler requires the user to apply a compressive force to the closed end of the canister. The internal components of the metering valve assembly are spring loaded so that, typically, a compressive force of between 15 and 30 N is required to activate the device. In response to this compressive force, the canister moves axially with respect to the valve stem by a distance sufficient to actuate the metering valve and causes a metered quantity of the drug and propellant to be expelled through the valve stem. This is then released into the mouthpiece *via* a nozzle in the stem block. A user inhaling through the drug delivery outlet of the device at this point will thus receive a dose of the drug.

The use of metered dose inhalers has become increasingly widespread in recent years owing to the fact that they enable the patient to administer an accurate dose of medicament when required. This is particularly useful for patients whose respiratory difficulties manifest themselves suddenly.

One of the problems most frequently encountered with the standard metered dose inhaler described as above is that the patient has difficulty co-ordinating the action of depressing the aerosol canister with the act of inhalation. This can be particularly problematic when the patient is in distress due to a sudden onset of respiratory difficulties. It is also difficult for small children to co-ordinate these actions properly. Such problems tend to undermine the effectiveness of self-administration. These difficulties have now been overcome by the introduction of breath-operated actuators such as those described in the Applicants' International Patent Application No. WO 92/09323.

When a pressurised inhaler is operated, the propellant stream explosively breaks up at atmospheric pressure to produce a range of different sizes of propellant droplets containing the drug, either in suspension or possibly in solution. Although the propellant in the droplets tends to evaporate, causing the droplets to decrease in size with time, the patient still receives a cloud of drug-containing droplets of various sizes.

The droplets tend to separate according to their sizes under the influence of gravitational forces and air currents. The smaller droplets, typically less than 5 - 6 µm in diameter, pass through the patient's oro-pharynx to enter the trachea, bronchi and lower airways where they are able to exert a therapeutic effect.

Larger droplets, on the other hand, may be deposited in the patient's oro-pharynx, owing to their different inertial characteristics, having escaped impingement on the standard actuator mouthpiece. Such oro-pharyngeal deposition is undesirable for a number of reasons. For example, the patient may experience an unpleasant taste, or a cooling effect as propellant droplets are rapidly evaporated by contact with warm mucosa. Unwanted deposition of certain classes of compounds may even cause undesirable local side effects, such as the corticosteroids which may lead to *Candida* infections ("thrush").

One successful approach to the elimination of problems caused by the deposition of large droplets in unwanted areas is the use of so-called "large volume spacers". These are reservoirs having a typical capacity of around 750 ml which are adapted at one end to be attached to the delivery outlet of a pressurised inhaler, with a mouthpiece at the other end for engagement with the user's mouth. Usually, the large volume spacer is equipped with a one-way valve to prevent user exhalation into the spacer cavity. In use, the inhaler is actuated into the spacer cavity, where the emitted aerosol is effectively trapped. Large droplets may either impinge on the spacer walls or fall to the floor of cavity. The patient inhales the aerosol from the spacer cavity, the inhaled cloud consisting of predominantly fine droplets which pass with the inhaled air stream into the lungs by virtue of their relative aerodynamic stability.

The patient need not inhale simultaneously with inhaler actuation; some delay is possible. Thus, the need for precise co-ordination of actions is removed and some of the problems associated with patient-controlled instantaneous drug administration are alleviated. However, the means of using large volume spacers has not been optimised and variation in practical use is possible.

Unfortunately, the use of large volume spacers introduces a different range of difficulties. Foremost among these is the sheer size of a large volume spacer. Such a device cannot be carried conveniently in the pocket or in a handbag, so the advantage of portability which makes metered dose inhalers so attractive is immediately cancelled out by the large volume spacer requirement. Also, it is not always possible for particularly frail persons or very small children to manipulate the inhaler/spacer combination. Such patients therefore require assistance which again undermines the convenience of the inhaler device.

Attempts have been made to solve the above problems with collapsible telescopic spacers, but these have met with only limited success. In any case, they do not satisfactorily overcome the co-ordination problem described earlier.

So-called "tube" spacers have been proposed as an alternative but have enjoyed only limited success. Whilst they are more compact, their performance as holding chambers is ineffective because virtually all of the aerosol is deposited on the walls of the spacer within a very short time. Tube spacers merely trap some of the large droplets which would otherwise be deposited in the patient's oro-pharynx and the co-ordination problem remains unsolved. Hitherto, the only effective way of using a tube spacer/pressurised inhaler combination has required co-ordination of inhaler activation with patient inhalation. As explained above, such coordinated action is not easy for every category of patient.

International Patent Application No. WO 93/18811 discloses a dry powder inhaler apparatus equipped with a separator containing one or several baffle plates. These baffle plates act to break up large particles of dispensed medicament by impaction and thereby increase the inspired medicament dose.

European Patent Application No. 0 504 459 discloses an atomiser apparatus for respiratory tract treatment. the atomiser has a cylindrical atomising chamber with inlet and outlet ports for the fluid mist. At least one of the ports is formed so as to generate a spiral vortex around the longitudinal axis of the chamber.

It is an object of the present invention to solve the aforementioned co-ordination problem whilst facilitating patient-administration of an effective dose of the required drug by means of a compact inhaler system.

The invention is a breath-operated inhaler device comprising an actuator body, a pressurised medicament dispenser containing medicament in suspension or in solution in propellant means, and medicament delivery outlet means through which medicament is delivered in response to inhalation by a user, wherein said actuator body actuates said medicament dispenser to deliver a metered dose of medicament entrained in propellant in response to user inhalation, characterised in that said medicament delivery outlet means is provided with an optimisation chamber equipped with a mouthpiece, and in that said chamber has a volume in the range from 20 ml to 200 ml.

Such an arrangement confers the advantage of the large volume spacer (no requirement for co-ordination of inhaler activation with patient inhalation) by facilitating drug delivery in response to patient inhalation. Moreover, the advantages of the tube spacer are also present because the inventive arrangement is compact but still allows large droplets/particles to be retained in the aerosol optimisation chamber. As indicated above, the drawback of tube spacers is avoided. There is no need for co-ordinated inhaler activation and patient inhalation.

In the invention, the medicament dispenser is a pressurised container containing the medicament in suspension or in solution in a propellant, and the actuator body is designed to actuate the pressurised container in response to inhalation on the part of the user.

Conveniently, the optimisation chamber is a separate device which can be engaged by a push-fit connection to the mouth-piece of a conventional breath-operated inhaler device by the patient. In an especially preferred form, the actuator body and the optimisation chamber are pivotally connected to each other to form a unitary device. In such an arrangement, the optimisation chamber may be designed to serve as a cover for the medicament delivery outlet of the device, thereby ensuring that the air passageway of the device is protected from dirt or other foreign body ingress. Deployment of the optimisation chamber then opens up a passageway from the medicament delivery outlet of the actuator body to the patient's mouth *via* the chamber interior.

Surprisingly, it has been found that the apparatus according to the present invention maintains a high respirable fraction of the delivered dose of medicament. In relation to breath-operated inhaler devices used without any type of spacer, apparatus according to the present invention delivers a respirable fraction which is at least as great. In relation to standard inhalers used in combination with large volume spacers, apparatus according to the present invention delivers a significantly higher respirable fraction.

The invention will now be described by way of example only with reference to the drawings, in which:
- Figure 1: is a schematic cross-sectional view through one embodiment of apparatus according to the present invention;
- Figure 2: is a graph comparing performance of a breath-operated inhaler device used on its own, a standard inhaler used with a large volume spacer and a device in accordance with the present invention for a 100 µg medicament dose, and
- Figure 3: is a graph similar to Figure 2, comparing performance for a 250 µg medicament dose.

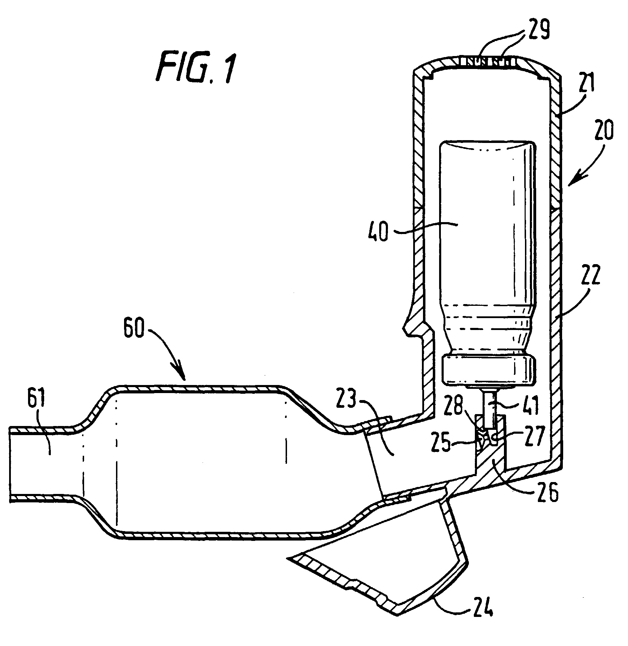

Referring now to Figure 1, an embodiment of inhaler apparatus according to the present invention is shown which comprises an actuator body 20, a pressurised medicament dispenser 40 and an aerosol optimisation chamber 60. The actuator body 20 is formed of a top section 21 and a bottom section 22, the bottom section 22 having an integrally-formed mouthpiece 23 and a pivotally-mounted dust cover 24 for preventing ingress of dust through the mouthpiece when the apparatus is not in use. The top section 21 accommodates the mechanism for effecting breath-actuation, but in view of the fact that the precise method by which breath-actuation is achieved does not form part of the present invention, this feature will not be described in detail here. As shown, top section 21 includes a series of air inlets 29 for admitting air to the apparatus in response to patient inhalation.

As shown, the actuator body 20 houses a pressurised medicament dispenser 40 in the form of aerosol canister of generally cylindrical shape. The aerosol canister has a stem 41 which contains an aerosol dispensing valve (not shown) of conventional design. A pedestal 26 formed on the base of the actuator body 20 is provided with a bore 27 dimensioned to form an airtight seal with the stem 41 of the aerosol canister. A shoulder 28 in the bore forms a seat for the tip of the stem 41 and helps to maintain the aerosol canister in position within the actuator body 20. Nozzle 25 communicates between the bore 27 and the mouthpiece 23.

The apparatus is shown with the dust cover 24 pivoted to the open position to allow attachment of aerosol optimisation chamber 60 to the mouthpiece 23 of the actuator body 20. The optimisation chamber 60 has its own mouthpiece formation 61 at the end thereof remote from the connection to the actuator body 20.

In use, the patient inhales through the mouthpiece 61 of the optimisation chamber. In response to patient inhalation, the actuation mechanism of the actuator body 20 moves the aerosol canister so that its dispensing valve is opened, thereby delivering a measured dose of medicament entrained in propellant issues through nozzle 25. The measured dose expands explosively into a cloud of droplets of various sizes which pass into the aerosol optimisation chamber 60. Here, large droplets (typically of diameter greater than 6 - 7 µm) impinge upon the chamber walls and are not inhaled by the patient. Smaller droplets are relatively aerodynamically stable and are inspired by the patient. A high proportion of the stabilized cloud is drawn into the patient's trachea, bronchi and lower airways where the drug is able to exert its therapeutic effect.

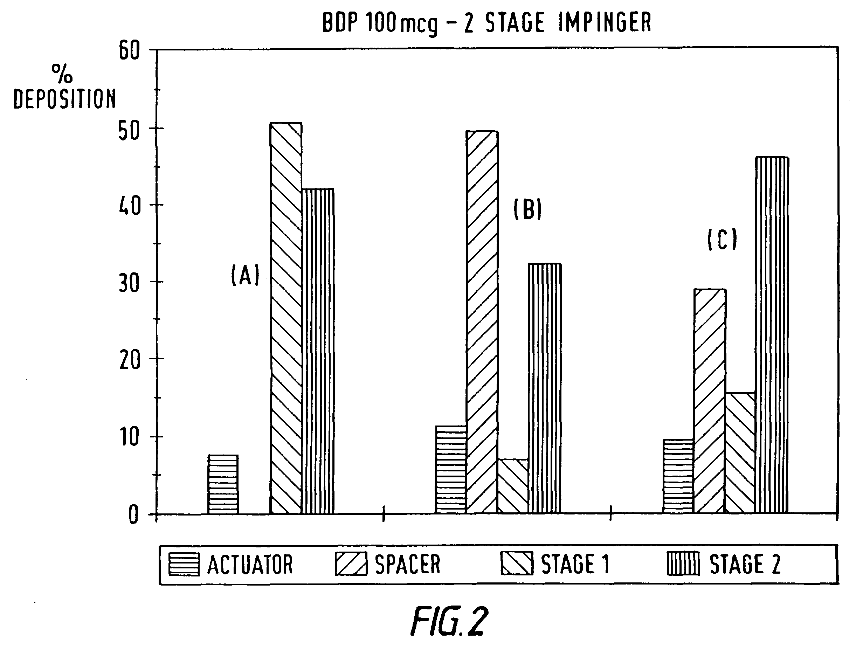

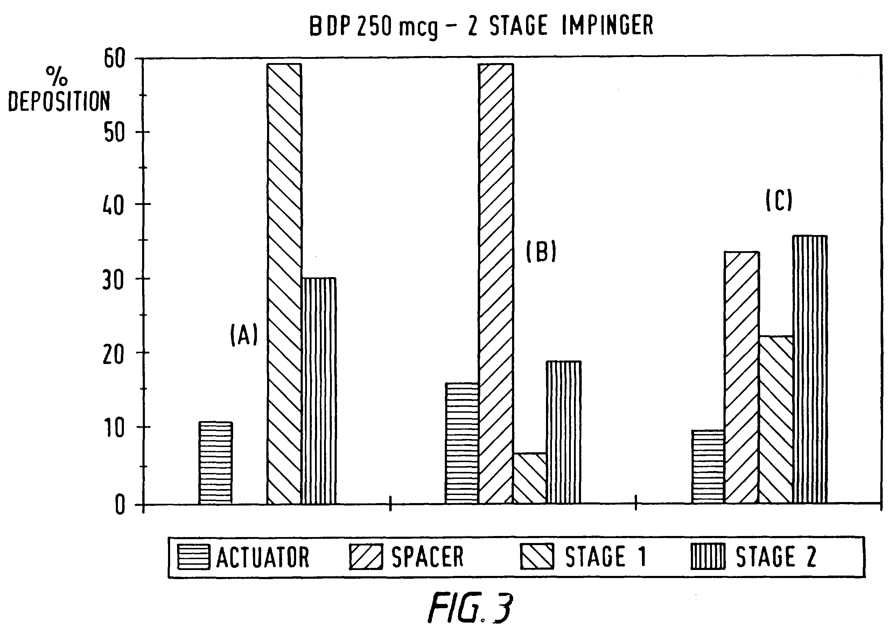

Turning now to Figures 2 and 3, these show comparative performances for various arrangements of inhaler apparatus. The Figure 2 graph compares performance for a 100 µg dose of beclomethasone dipropionate (BDP) dispensed through:
(A) a breath-operated inhaler device used on its own;
(B) a standard inhaler used with a large volume spacer, and
(C) an apparatus in accordance with the present invention.

The so-called "Stage 1" fraction is the fraction containing the larger droplets of diameter 6 -7 µm or greater. The "Stage 2" fraction is composed of finer droplets and is generally regarded by persons skilled in the art as the respirable fraction, that is the proportion of an inhaler cloud most likely to be associated with therapeutic activity. The Stage 1 and Stage 2 fractions were differentiated by a two-stage impinger apparatus specified in the British Pharmacopoeia 1994, which can be used to separate an aerosol cloud on the basis of droplet size.

It will be noted that Figure 2 shows that, with the breath-operated inhaler device used on its own, the Stage 1 fraction amounts to 50% of the delivered dose. The standard inhaler combined with a large volume spacer has a Stage 1 fraction of less than 10%, whilst the apparatus according to the present invention shows a Stage 1 fraction of around 15%, both the latter showing what is likely to be clinically useful reductions in oro-pharyngeal deposition.

However, with reference to the critical Stage 2 fraction, it can be seen that the breath-operated inhaler device delivers a respirable fraction of roughly 40% of the measured dose. The apparatus according to the present invention also shows a high respirable fraction (>45%), whereas the standard inhaler/large volume spacer combination shows a respirable fraction of just over 30%. For some medicaments, this would be below the minimum threshold permitted by the British Pharmacopoeia. It should also be borne in mind that the respirable fraction obtained from a large volume spacer diminishes with time, so this figure of 30% represents the maximum respirable fraction available to the patient assuming immediate inhalation.

The Figure 3 graph shows a corresponding analysis for a 250 µg dose of beclomethasone dipropionate. This graph shows that the trends observed for a 100 µg are more sharply accentuated at higher doses. Not surprisingly, the unadulterated breath-operated inhaler device shows a high Stage 1 fraction, but delivers a respirable fraction of only 30%. The Stage 1 fraction for the standard inhaler/large volume spacer combination is little more than 5%, but the respirable fraction is significantly reduced to a level of less than 20%. By contrast, the apparatus according to the present invention not only maintains a low Stage 1 fraction (21%), but also delivers a respirable fraction of 35%.

These figures show that the apparatus according to the present invention gives rise to surprising synergistic effects which are likely to result in significant patient benefits. The fact that the apparatus uses a breath-operated inhaler device means that it is capable of providing an accurate and consistent injection of dose when the patient inhales. The dose injection can be controlled by the mechanical design of the breath-operated inhaler device, for example to occur at an inhaled air flow rate of 25 l/min. Such actuation is totally automatic and reproducible in response to patient inhalation.

Moreover, the aerosol optimisation chamber which fits onto the mouthpiece of the actuator body is of a sufficiently small volume to ensure that air flow is immediately transmitted to the actuating mechanism of the apparatus. Also, the volume of inhalable air carrying the medicament dose is within the restricted inhaled air volume of an asthmatic patient (perhaps 500 ml).

In conclusion, an apparatus designed in accordance with the present invention is capable of removing a significant proportion of the large droplets of medicament-containing propellants whilst maintaining the critical respirable fraction of the aerosol which contains the therapeutically active portion of the administered medicament. Both of these objects are achieved without requiring the patient to coordinate inhaler activation with inhalation.

Although the invention has been particularly described with reference to only one simple embodiment, it will be understood that various modifications are possible within the scope of the claims.

## Claims

1. A breath-operated inhaler device comprising an actuator body (20), a pressurised medicament dispenser (40) containing medicament in suspension or in solution in propellant means, and medicament delivery outlet means (23) through which medicament is delivered in response to inhalation by a user, wherein said actuator body (20) actuates said medicament dispenser (40) to deliver a metered dose of medicament entrained in propellant in response to user inhalation, characterised in that said medicament delivery outlet means (23) is provided with an optimisation chamber (60) equipped with a mouthpiece (61), and in that said chamber (60) has a volume in the range from 20 ml to 200 ml.

2. A breath-operated inhaler device as claimed in claim 1 wherein the optimisation chamber (60) is a separate device engageable by a push-fit connection to said drug delivery outlet means (23).

3. A breath-operated inhaler device as claimed in claim 1 or claim 2 wherein the actuator body (20) and the optimisation chamber (60) are pivotally connected to each other.

4. A breath-operated inhaler device as claimed in any preceding claim wherein the optimisation chamber (60) serves as a dust cover for the drug delivery means (23).

## Patentansprüche

1. Atembetriebene Inhaliervorrichtung, umfassend ein Betätigungsteil (20), einen unter Druck stehenden Medikamentspender (40) enthaltend das Medikament in Suspension oder Lösung in Treibhilfsmitteln, und ein medikamentzuführendes Auslaßmittel (23), durch welches das Medikament in Erwiderung auf die Inhalation durch einen Benutzer befördert wird, wobei das Betätigungsteil (20) den Medikamentspender (40) betätigt, um eine abgemessene Menge des im Treibmittel mitgerissenen Medikaments in Erwiderung auf die Inhalation des Benutzers zu befördern,
**dadurch gekennzeichnet,** daß das medikamentzuführende Auslaßmittel (23) mit einer Optimierungskammer (60) versehen ist, die mit einem Mundstück (61) ausgestattet ist, und daß die Kammer (60) ein Volumen im Bereich von 20 ml bis 200 ml aufweist.

2. Atembetriebene Inhaliervorrichtung wie in Anspruch 1 beansprucht, wobei die Optimierungskammer (60) eine separate Vorrichtung ist, die mittels einer Schubpassungsverbindung (push-fit-connection) an dem Arzneimittelbeförderungs-Auslaßmittel (23) einrastbar ist.

3. Atembetriebene Inhaliervorrichtung wie in Anspruch 1 oder Anspruch 2 beansprucht, wobei das Betätigungsteil (20) und die Optimierkammer (60) auf drehbare Weise miteinander verbunden sind.

4. Atembetriebene Inhaliervorrichtung, wie in einem der vorhergehenden Ansprüche beansprucht, wobei die Optimierkammer (60) als Abdeckhaube für das Arzneimittelbeförderungsmittel (23) dient.

## Revendications

1. Un dispositif inhalateur réagissant à la respiration, comprenant un organe d'actionnement (20), un distributeur de médicament sous pression (40) contenant un médicament en suspension ou en solution dans des moyens de propulseur, et des moyens de sortie pour l'administration de médicament (23) au moyen desquels le médicament est administré en réponse à l'action d'inhalation d'un utilisateur, dans lequel ledit organe d'actionnement (20) actionne ledit distributeur de médicament (40) pour administrer une dose mesurée de médicament entraînée dans le propulseur en réponse à une action d'inhalation d'un utilisateur, caractérisé en ce que lesdits moyens de sortie pour l'administration de médicament (23) sont pourvus d'une chambre d'optimisation (60) équipée d'une embouchure (61), et en ce que ladite chambre (60) présente un volume dans la plage de 20 ml à 200 ml.

2. Un dispositif inhalateur réagissant à la respiration selon la revendication 1, dans lequel la chambre d'optimisation (60) est un dispositif séparé susceptible d'être engagé par une liaison d'emmanchement à frottement, sur lesdits moyens de sortie pour l'administration de médicament (23).

3. Un dispositif inhalateur réagissant à la respiration selon la revendication 1 ou la revendication 2, dans lequel l'organe d'actionnement (20) et la chambre d'optimisation (60) sont reliés de manière pivotante l'un avec l'autre.

4. Un dispositif inhalateur réagissant à la respiration selon l'une quelconque des revendications précédentes, dans lequel la chambre d'optimisation (60) sert de couvercle anti-poussière pour les moyens d'administration de médicament (23).
